# FASCICULE DE BREVET EUROPEEN

(11) **EP 0 499 527 B1**
(45) Date de publication et mention de la délivrance du brevet: **02.05.1997**
(21) Numéro de dépôt: 92400351.0
(22) Date de dépôt: 11.02.1992
(51) Int. Cl.: C07D 491/14, C07D 471/14, C07D 405/04, C07D 495/14

(54) **Composés dérivés des beta-carbolines ligands du récepteur des benzodiazépines ayant un effet agoniste inverse et antagoniste vis-à-vis des benzodiazépines et médicaments les contenant**
Beta-carbolinderivate, Ligande für Benzodiazepinrezeptoren mit agonistischer Inverse und Benzodiazepinantasonistische Wirkungen und diese enthaltende Arzneimittel
Beta-carboline derivatives, ligands of benzodiazepine receptors with inverse agonist and benzodiazepine antagonist effects and medicaments containing them

(30) Priorité: 12.02.1991 FR 9101595
(43) Date de publication de la demande: 19.08.1992
(73) Titulaire: CENTRE NATIONAL DE LA RECHERCHE SCIENTIFIQUE (CNRS), 75794 Paris Cédex 16 (FR)
(72) Inventeur: Dodd, Robert, F-75009 Paris (FR); Potier, Pierre, F-75007 Paris (FR); Rossier, Jean, F-75016 Paris (FR); Dorey, Gilbert, F-92370 Chaville (FR); Dubois, Laurent, F-91190 Gif-sur-Yvette (FR); Prado de Carvalho, Lia, F-75013 Paris (FR)
(74) Mandataire: Warcoin, Jacques

(56) Documents cités:
- EP-A- 0 237 467
- European Journal of Pharmacology, vol. 76, 1981, pages 281-284
- Journal of Medicinal Chemistry, vol. 32, 1989, pages 1989-1802
- Heterocycles, vol. 20, 1983, pages 1295-1313

## Description

L'invention se rapporte à des nouveaux composé dérivés des β-carbolines ligands du récepteur des benzodiazépines ayant un effet agoniste inverse et antagoniste vis-à-vis des benzodiazépines, et aux compositions pharmaceutiques contenant ces composés.

Elle concerne également les procédés de préparation desdits composés et les nouveaux composés éventuellement utilisables pour mettre en oeuvre ces procédés de préparation.

Les benzodiazépines-1,4 (par exemple, le Valium) constituent une classe de médicaments largement prescrits à cause de leurs activités anxiolytique, anticonvulsivante, sédative-hypnotique et myorelaxante. Le mécanisme d'action des benzodiazépines est resté longtemps méconnu jusqu'à la mise en évidence de sites de fixation (ou "récepteurs") spécifiques pour ces molécules sur les membranes neuronales du système nerveux central. L'importance physiologique de ces récepteurs a été démontrée par l'existence d'une bonne corrélation entre, d'une part, la valeur de l'affinité des différentes benzodiazépines pour le récepteur (mesurée par déplacement d'une benzodiazépine radioactive) et, d'autre part, leur efficacité thérapeutique.

De même, on a trouvé que certaines β-carbolines se comportaient comme agonistes du récepteur et des benzodiazépines.

Ainsi, l'Abécarnil, développé par les laboratoires Schering comme anxiolytique, 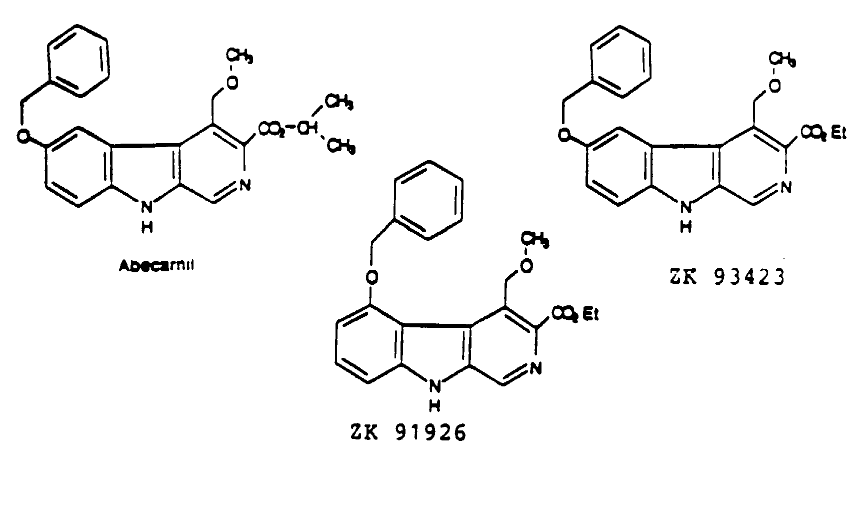 est en cours d'essais cliniques. D'autres β-carbolines apparentées telles que le ZK 93423 et le ZK 91296 possèdent également des propriétés analogues à celles du diazépam in vivo (anxiolytique, anticonvulsivante...).

En recherchant un ligand endogène du récepteur des benzodiazépines, Braestrup et coll. (Proc. Natl. Acad. Sci., USA, 77, 2288-2292, 1980) ont mis en évidence une molécule, l'ester éthylique de l'acide β-carboline-3-carboxylique (la β-CCE,), extraite de l'urine humaine, qui se fixe avec une très bonne affinité aux récepteurs centraux des benzodiazépines (CI₅₀ = 4nM, in vitro, cerveau de rat). Par Par contre, la β-CCE possède in vivo des effets pharmacologiques opposés à ceux des benzodiazépines. Ainsi, la β-CCE est proconvulsivante chez la souris, facilitant les convulsions provoquées par d'autres agents tels que le pentylènetétrazole.

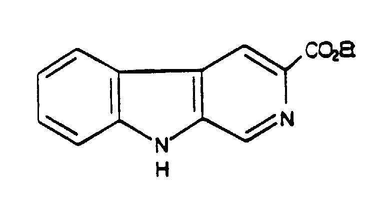

Le terme "agoniste inverse" est maintenant utilisé pour désigner ces ligands du récepteur des benzodiazépines qui possèdent des activités complètement ou partiellement opposées à celles des benzodiazépines.

L'intérêt thérapeutique des β-carbolines se situe à plusieurs niveaux. Ainsi, l'équipe, auteur de la présente invention, a été la première à démontrer que de telles molécules (par exemple, la β-CCM ci-dessous), 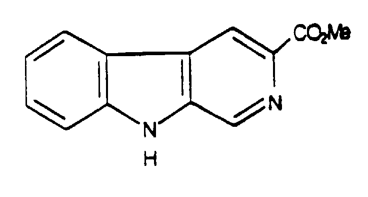 agonistes inverses du récepteur des benzodiazépines, ont un effet positif sur l'apprentissage et la mémorisation (Venault et coll., Nature, 321, 864-866, 1986). Bien que la β-CCM n'ait pu être testée chez l'homme pour ses effets promnésiants dus à son activité fortement convulsivante, d'autres analogues (par exemple, le ZK 93426, ont montré leur aptitude à améliorer l'apprentissage et la rétention des informations chez l'homme (Duka et coll. Psychopharmacology, 93,421-427,1987)

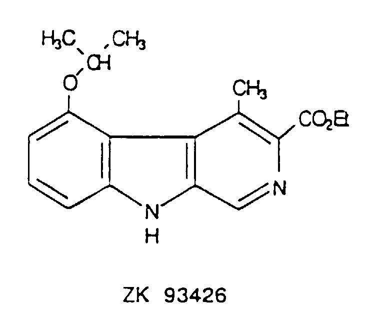

Il a également été décrit des β-carbolines comportant un cycle γ-ou δ-lactone ou γ - ou δ-lactame. Ces composés présentent une affinité moyenne pour le récepteur des benzodiazépines (Neef G. et coll. J. Chem. Soc. Chem. Comm., 366, 1982 et Neef, G. et coll. Heterocycles, 20, 1295-1312, 1983).

D'autres dérivés d'hétéroaryl-oxy-β-carboline utiles en tant que médicaments du système nerveux central ont été décrits dans le document EP-A-237 467.

Dorey et al, Journal of Medicinal Chemistry, 1989, 32, No. 8, ont décrit d'autres avantages analogues des 3-carboxy-β-carbolines et leur affinité pour le récepteur de la benzodiazépine.

Robertson et al, European Journal of Pharmacology, 76 (1981) 281-284 ont également décrit l'interaction de certaines β-carbolines avec le récepteur de la benzodiazépine.

En conséquence, un des buts de la présente invention est de proposer de nouveaux composés présentant une affinité avec le récepteur des benzodiazépines tout en ayant un effet agoniste inverse et antagoniste.

Un autre but de la présente invention est de proposer de nouvelles compositions thérapeutiques utiles notamment dans le traitement de troubles nerveux.

Un autre but de la présente invention est de proposer de nouvelles compositions thérapeutiques ayant un effet positif sur l'apprentissage et la mémorisation et pouvant être utilisables pour le traitement des problèmes cognitifs comme par exemple, dans la maladie d'Alzheimer.

L'invention concerne donc en premier lieu les composés de formules: 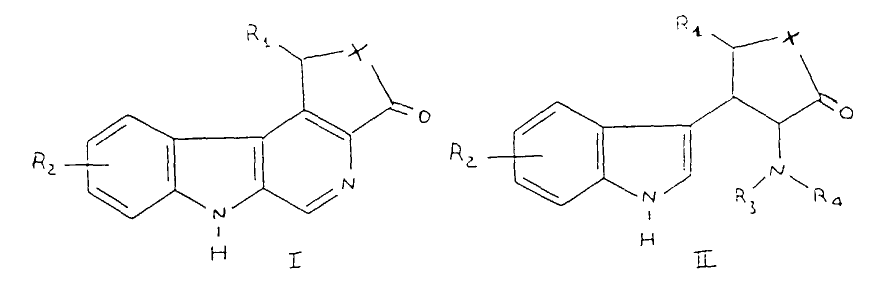 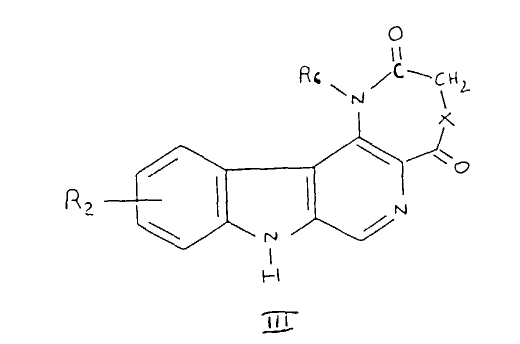
X est l'atome d'oxygène, de soufre ou un radical divalent NR₅,
R₁ est choisi Parmi les radicaux alkyl substitués en α par un radical hydroxy ou alcoxy,
R₂ est un atome d'hydrogène ou un radical choisi parmi les radicaux alkyl, hydroxy, alcoxy, acyloxy, benzyloxy,
R₃, R₄, R₅ et R₆ sont choisis parmi l'atome d'hydrogène ou le radical alkyle éventuellement substitué par un radical hydroxy, alcoxy, alkoxycarbonylméthyle,
ainsi que les tétrahydro dérivés de β-carboline correspondants et les sels ou dérivés d'ammonium quaternaires de ces composés pharmaceutiquement acceptables.

Dans la présente description, les radicaux alkyle, alkoxy ou acyloxy ont de préférence de 1 à 6 atomes de carbone et peuvent être linéaires ou ramifiés (de préférence linéaires).

Par ailleurs, certains composés présentent un ou plusieurs centres d'asymétrie. Ils peuvent donc exister sous une ou plusieurs formes optiquement actives. Dans ce cas, l'invention comprend lesdites formes optiquement actives de ces composés.

Parmi les sels, on peut citer : les chlorures, les chlorhydrates, tartrates, fumarates.

Selon une premiere variante préférée, les composés selon l'invention répondent à la formule I dans laquelle :
R₁ et R₂ ont la même signification que précédemment et X est l'atome d'oxygène ou un radical divalent N - R₅. R₂ est l'atome d'hydrogène. Encore plus avantageusement, R₁ est un radial hydroxyméthyle.

Selon une seconde variante préférée, les composés répondent à la formule II dans laquelle :
R₁ et R₂ ont la même signification que précédemment,
R₃, R₄ sont l'atome d'hydrogène et X est l'atome d'oxygène ou un radical divalent N - R₅. De préférence R₂ est l'atome d'hydrogène. Encore plus avantageusement, R₁ est un radical hydroxyméthyle.

Selon une troisième variante préférée, les composés répondent à la formule : 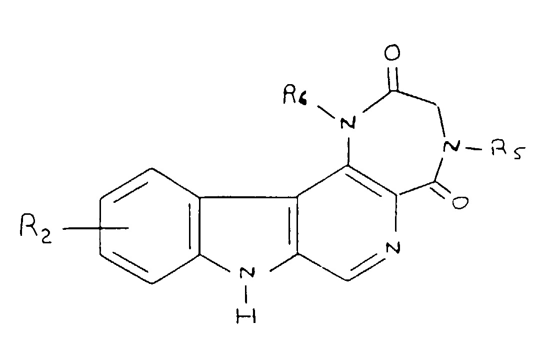 dans laquelle :
R₂, R₅, R₆ ont la même signification que précédemment indiqué.

De préférence R₅, R₆ sont choisis parmi l'atome d'hydrogène ou un radical alkyle et/ou R₂ est l'arome d'hydrogène.

Selon une quatrième variante, l'invention concerne les composés de formule (I) tétrahydro des premières et troisièmes variantes.

De préférence, les composés selon l'invention sont choisis parmi les composés suivants sous forme optiquement active ou racémique :
- Dihydro-1,12 méthyl-2 indolo [3',2':4,5] pyrido [2,3-f] 1H, 3H, 6H diazépine-1,4 dione-3,12 ;
- Hydroxyméthyl-10 butanolide (2,3-b)-β-carboline,
- 3-hydroxyméthyl-4-(5-benzyloxy-3-indolyl)-5-amino-gamma-lactone.

L'invention concerne également les médicaments consistant en un des composés selon l'invention, tels qu'ils viennent d'être décrits ci-avant et les compositions pharmaceutiques contenant au moins un de ces médicaments et un support acceptable. Ces médicaments et compositions sont utiles pour le traitement médical ou vétérinaire de certains troubles liés au fonctionnement du système nerveux.

Ces médicaments et compositions sont particulièrement destinés à être utilisés en combinaison avec des benzodiazépines afin de compenser les effets néfastes dus à l'emploi de ces dernières. Ces composés eux-mêmes favorisent également l'apprentissage et la rétention d'information chez l'homme.

Ces médicaments et compositions peuvent ainsi être avantageusement destinés à participer à une thérapie efficace contre les maladies dégénératives du système nerveux en particulier des démences du type Alzheimer.

Les compositions pharmaceutiques sont notamment formulées pour être ingérées oralement ou pour être injectées. Néanmoins, d'autres présentations peuvent également être envisagées dans le cadre de la présente invention.

La posologie dépendra pour partie de la maladie à traiter ainsi que de sa gravité et également du type de l'individu (poids, âge).

Il pourra être avantageusement envisagé une posologie allant de 0,1 mg/kg à 20 mg/kg.

L'invention concerne également les procédés de préparation permettant d'obtenir les composés selon l'invention.

Un procédé de préparation de composés de formule I dans laquelle X est l'atome d'oxygène, R₂ a la même signification que précédemment, R₁ est un radical méthyle substitué par un radical hydroxy ou alcoxy consiste à oxyder un composé de formule IV ou de formule VIII : 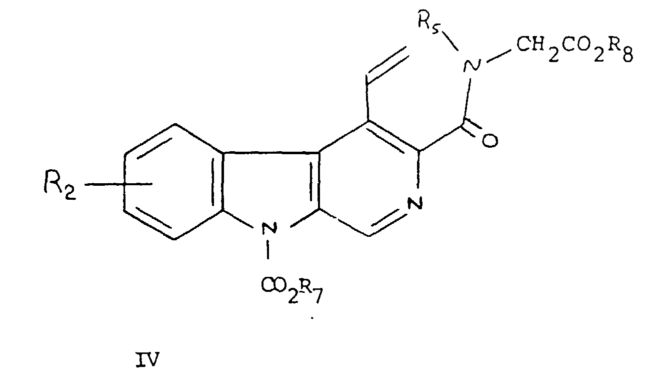
R₅ différent de H,
R₈ étant un radical C₁-C₆ alkyle 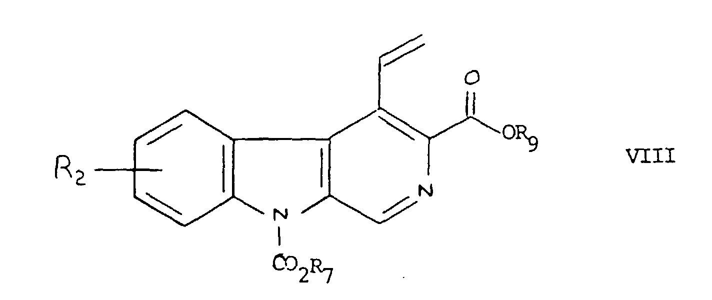
R₉ étant un radical alkyle, par exemple par le tétroxyde d'osmium, suivi par un déblocage de l'atome d'azote et éventuellement par une éthérification au moyen de l'alcool correspondant ou vice versa.

Le composé de formule IV peut être obtenu selon le schéma suivant: 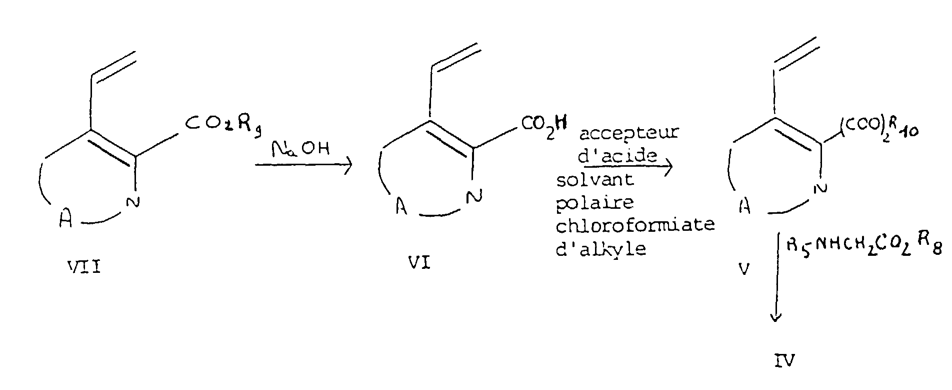
R₉, R₁₀ étant choisis parmi les radicaux alkyle,
A étant le reste du squelette β-carboline de I.

Ainsi, l'invention a également pour objet une préparation originale des composés de formule V et VI selon le schéma ci-dessus.

La synthèse du composé de formule VIII est obtenu par traitement du composé de formule VII (Neef et coll. Heterocycles 20, 1295 (1983) par un alkyle chloroformiste et de la triéthylamine dans le THF :

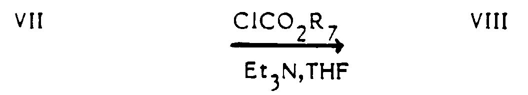

Un autre procédé consiste à faire réagir un composé de formule VII ou de formule : 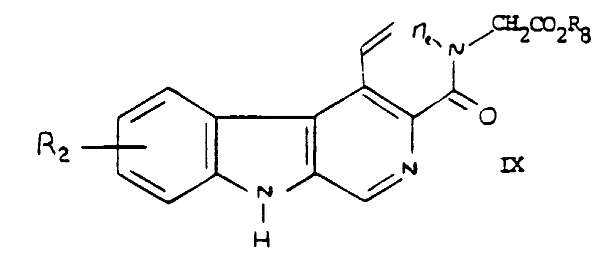 avec le tétroxyde d'osmium et éventuellement d'éthérifier au moyen de l'alcool correspondant.

Les composés de départ se préparent de la même façon que précédemment par exemple par déblocage de l'azote du noyau pyrrole.

Un procédé de préparation des composés de formule (I) dans laquelle X est un radical divalent N-R₅, R₁ est un radical hydroxy ou alcoxy consiste à transformer un composé de formule IV (avec R₅ est l'atome d'hydrogène) en composé de formule : 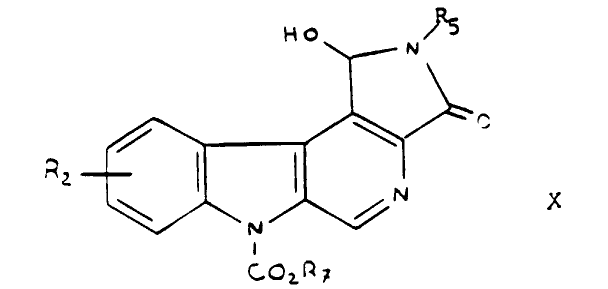 par ozonolyse, échange éventuel du groupe CH₂CO₂R₈ du lactame par un radical R₅ = alkyle, puis à débloquer l'atome d'azote du pyrrole par action d'un alcool en présence de sodium et puis éventuellement à éthérifier le composé obtenu.

Le composé de formule IV est obtenu comme indiqué précédemment.

Un procédé de préparation de composés de formule III, consiste à cycliser en milieu acide un composé de formule : 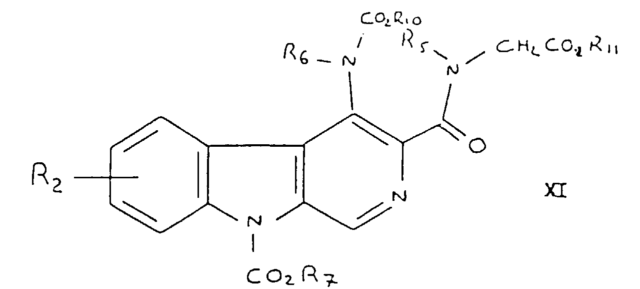 R₁₀, R₁₁ étant choisis parmi les radicaux alkyle (de préférence t-Bu pour R₁₀), puis à effectuer l'étape de déblocage selon le procédé précédent. Le produit de départ de formule XI peut être obtenu selon le schéma suivant: 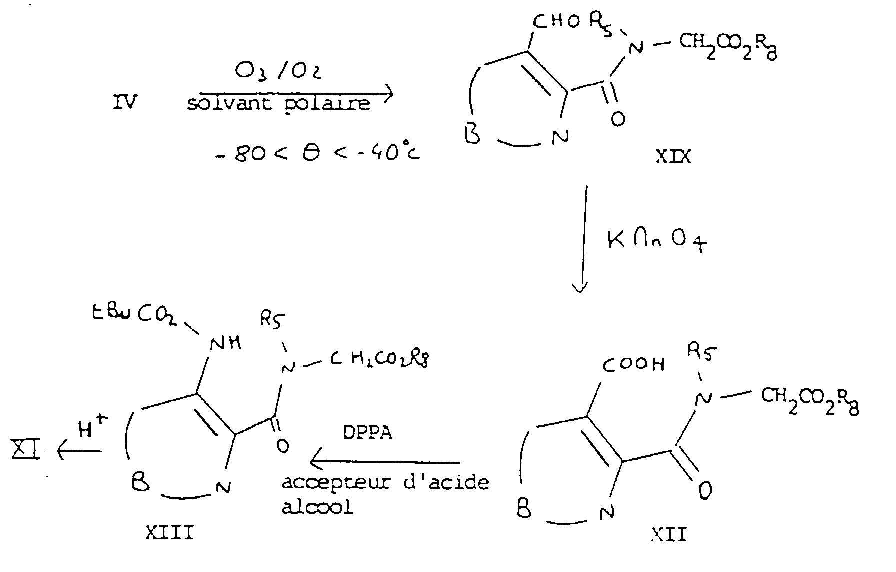 B étant le reste de β-carboline de IV.

La réaction de IV en XIX est analogue à celle conduisant au composé III et le plus souvent conduit à un mélange des deux espèces qu'une séparation subséquente permet de purifier.

Un procédé de préparation des composés de formule (II) dans laquelle X est l'atome d'oxygène, R₁ est un radical alkyle substitué en α par un radical hydroxy ou alcoxy, R₂, R₃, R₄ ont la même signification que précédemment consiste à chauffer un composé de formule : 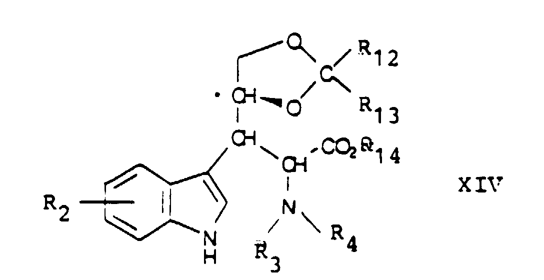 R₁₂, R₁₃, R₁₄ étant choisis parmi l'atome d'hydrogène ou les radicaux alkyle en milieu acétique aqueux.

Le composé de formule XIV peut être obtenu selon le procédé, donné à titre d'exemple, suivant :

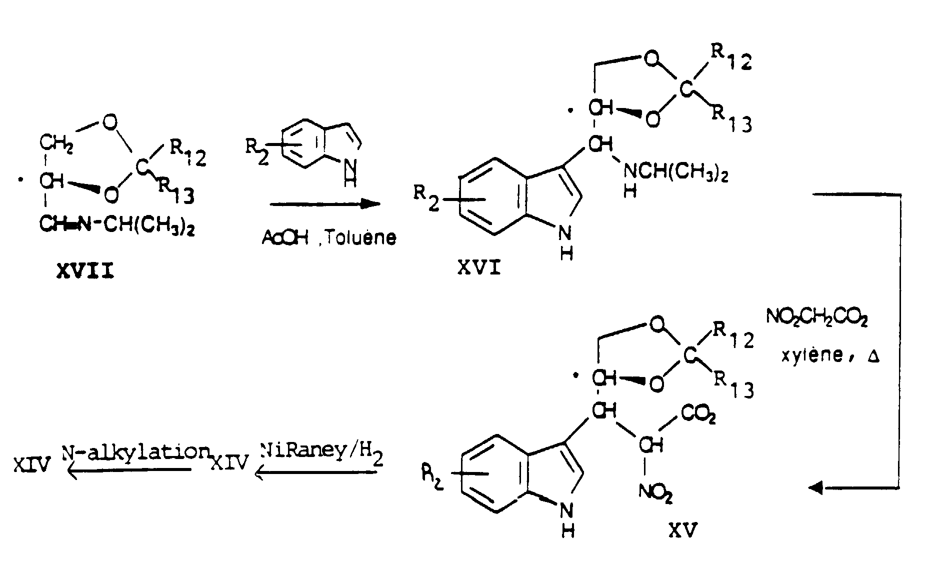

Un procédé de préparation des composés de formule (I) dans laquelle il s'agit des dérivés de β-carboline tétrahydro avec R₁ est un méthyle substitué par un hydroxy ou alcoxy et X = O consiste à faire réagir les composés de formule Il avec le formaldéhyde en présence d'un solvant polaire aprotique.

De même, il est possible d'atteindre ces composés en faisant réagir les composés de formule : 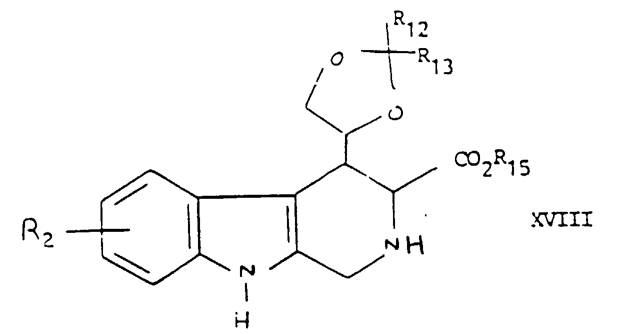 R₁₅ étant un radical alkyle en milieu acétique aqueux.

Les composés de formule XVIII sont obtenus par réaction du formaldéhyde, dans le toluène en chauffant, avec le composé de formule XIV (où R₃, R₄, sont l'atome d'hydrogène).

Il est également possible d'obtenir les composés de formule (I) où X = O, et R₁ est un radical alkyle substitué en α par un radical hydroxy par aromatisation du composé de formule XVIII précédemment décrit puis cyclisation en milieu acétique aqueux. On peut également atteindre cette famille de composés par aromatisation des composés tétrahydro correspondant.

L'invention concerne également les procédés de préparation stéréosélectifs permettant d'obtenir des composés selon l'invention présentant un ou plusieurs carbones asymétriques.

Ces procédés sont particulièrement intéressants dans le cas des composés de formule (I) dans laquelle X = O, R₁ est un alcoxy, hydroxy, hydroxyméthyle, alcoxyméthyle.

On donne ci-après à titre d'exemple un tel procédé.

### SYNTHESE STEREOSELECTIVE DES ISOMERES R ET S DE L'HYDROXYMETHYL-10 BUTANOLIDE (2,3-b)-β- CARBOLINE

La synthèse est illustrée par la suite de réaction suivante :

L'aldimine réagit sur le noyau indolique en présence d'acide acétique à 5°C dans le toluène pour conduire à la gramine attendue (Schéma 4). Le déplacement du groupement isopropylamine est réalisé par action du nitroacétate d'éthyle dans le xylène à reflux . Le dérivé nitré est isolé avec un rendement de 75 % après chromatographie.

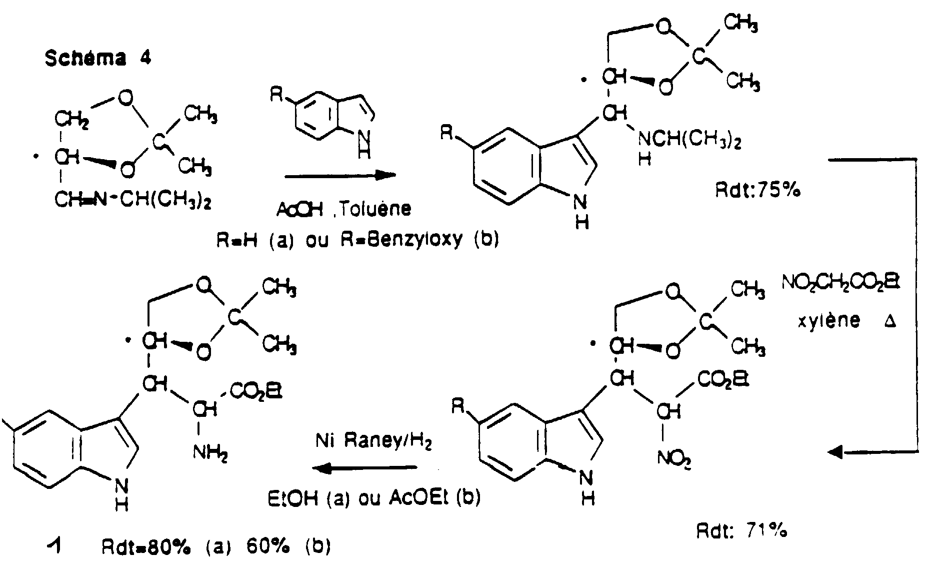

La réduction du groupement nitro a pu être effectuée en présence de nickel de Raney, sous atmosphère d'hydrogène dans l'acétate d'éthyle, sélectivement, sans débenzylation dans le cas du dérivé benzyloxy. Les amines résultant ont ensuite été cyclisées en aminolactone 2 ou transformées en tétrahydro-β-carboline 3 selon un mécanisme décrit connu (schéma 5).

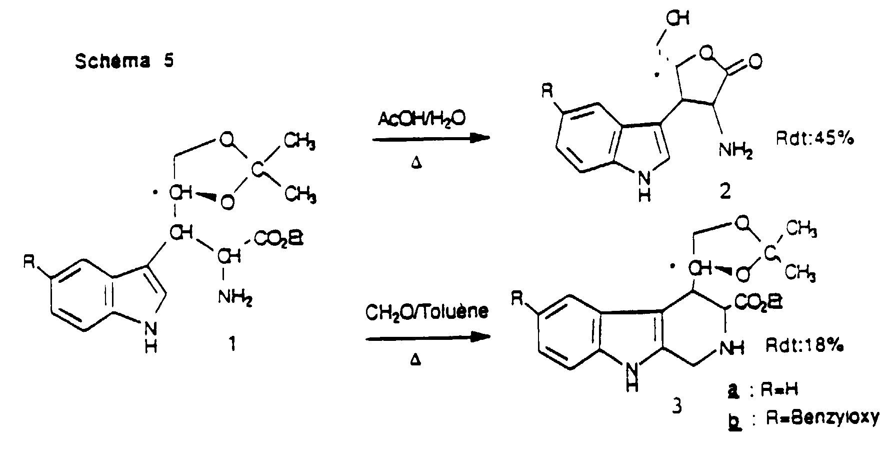

L'aminolactone 2 peut être transformée en β-carboline 4 selon des conditions douces connues.

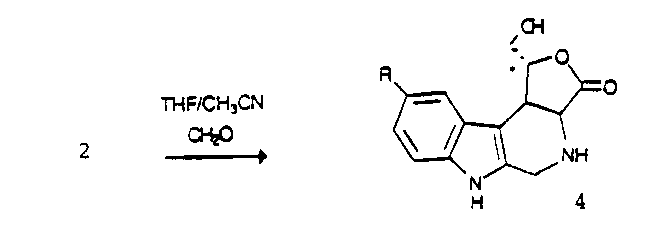

Les tétrahydro-β-carbolines 2 et 4 sont aromatisées en présence de soufre au reflux du DMSO. Dans le cas de 2, on obtient la β-carboline 3 que l'on cyclise en présence d'acide acétique et d'eau pour former les lactones souhaitées 5 (R =H) et 6 (R=OCH₂C₆H₅) de façon stéréosélective. La tétrahydro-β-carboline 4 donne, dans ces conditions, directement la lactone optiquement pure désirée (isomère R).

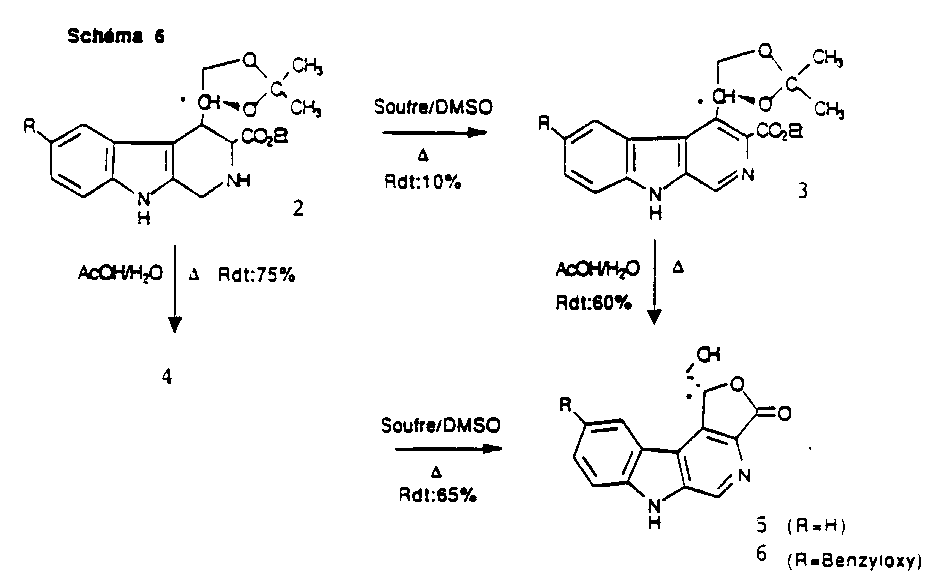

L'isomère S de l'hydroxyméthyl-10 butanolide (2,3-b)-β-carboline (5-5) a été obtenu à partir du L-glycéraldéhyde-acétonide 7 selon les voies de synthèse décrites ci-dessus. Le composé 7 est lui-même synthétisé à partir de l'acide L-ascorbique 8 selon un procédé "one-pot" (en une seule phase) connu.

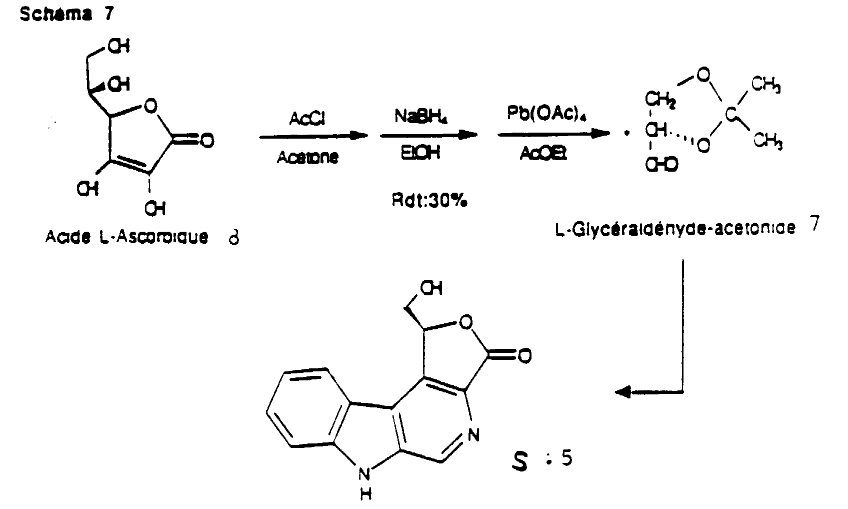

Certains autres composés, dont le procédé de préparation n'est pas explicitement décrit, sont obtenus de façon connue pour un homme de métier.

Les exemples qui suivent illustrent les procédés de préparation de quelques uns des composés selon l'invention.

### Exemple 1:

### Hydroxyméthyl-10 butanolide (2,3-b) β-carboline (composé n° 5 R,S)

On dissout 700 mg (1,71 mmol) du composé de formule IV précédent dans un mélange de dioxanne (75 ml) et d'eau (30 ml) et on y ajoute une quantité catalytique de tétroxyde d'osmium solide. Le mélange est agité pendant 30 mn puis on y introduit 2 g (9,35 mmol) de périodate de sodium. L'ensemble est placé sous agitation, à température ambiante et à l'abri de la lumière, pendant 21 heures. Les sels inorganiques formés en cours de réaction sont alors filtrés et rincés à l'aide de dioxanne. Le solvant est ensuite évaporé sous pression réduite, le résidu repris à l'acétate d'éthyle et lavé avec une solution saturée de chlorure de sodium. Après séchage sur sulfate de sodium, évaporation et purification sur colonne silice [dichlorométhane-éthanol (8:0,2)] on isole 20 mg (0,06 mmol) du composé protégé fondant entre 202 et 204°C (rendement 5 %), 382 mg (0,93 mmol) d'aldéhyde, qui est le N-éthyl-éthoxycarbonyl N-méthyl (éthoxycarbonyl-9 formyl-4) β-carboline carboxamide-3 fondant à 113-114°C, 88 mg (0,21 mmol) de produit de départ n'ayant pas réagi.

La γ-lactone obtenue (11,7mg, 0,036 mmol) est déprotégée en présence de 2,5 ml d'éthanol. 0,6 mg de sodium à température ambiante sous agitation, neutralisé avec une petite quantité d'acide acétique puis les réactifs et solvants sont évaporés sous pression réduite. Le résidu huileux est repris au dichlorométhane ; le précipité résultant est filtré puis rincé à l'eau pour éliminer les sels inorganiques. On isole après séchage 6,6 mg (0,026 mmol du composé n° 5). Rendement : 72 % ; F = 278-280°C avec décomposition.

### Exemple 2:

### Dihydro-1,12 méthyl-2 indolo [3',2':4,5] pyrido [2,3-f] 1H, 3H, 6H diazépine-1,4 dione-3,12, 10(composé de formule III dans laquelle R₂=R₆ = H; X = NCH₃)

### Etape 1 : Préparation du N-méthyl N-éthyléthoxycarbonyl-(éthoxy-carbonyl-9 acide carboxylique-4) β-carboline carboxamide-3.

On dissout 285 mg (0,70 mmol) de β-carboline de formule IV dans laquelle R₂ = H, R₅ = Me, R₇ = R₈ = Et dans un mélange de dichlorométhane (15 ml) et de méthanol (5 ml) anhydre. Le mélange réactionnel est refroidi à -78°C à l'aide d'un bain d'éthanol et de carboglace. On y introduit alors un mélange d'oxygène et d'ozone pendant 1 h 50 mn (disparition complète du produit de départ sur C.C.M.) puis on y fait buller un courant d'azote pendant 5 mn. On introduit ensuite 0,4 ml (3,38 mmol) de triméthylphosphite et on laisse l'ensemble au repos, pendant 1 heure à température ambiante. Après évaporation des solvants sous pression réduite, on purifie le résidu huileux de couleur jaune par chromatographie liquide à haute performance (silice greffée : méthanol-eau ; 7:3). On isole un solide beige (96 mg ; 0,29 mmol) qui est le composé N-éthoxycarbonyl méthoxy-10 butanolide (2,3-b) β-carboline fondant à 180°C avec décomposition (rendement 42 %) et un aldéhyde (15 mg, 0,036 mmol) qui est le N-éthyl-éthoxycarbonyl N-méthyl (éthoxycarbonyl-9 formyl-4) β-carboline carboxamide-3 fondant à 113-114°C, avec un rendement de 5 %.

Voie A : Oxydation à l'acide méthachloroperbenzoïque.

La formyl-4 β-carboline obtenue (27 mg; 6,57 mmol) est dissoute dans un mélange de tétrahydrofuranne et d'eau (5:1). La solution est portée à basse température à l'aide d'un bain d'eau glacée et placée sous courant d'azote. On introduit alors 14,5 mg d'acide méthachloroperbenzoïque dans le mélange réactionnel, et on place l'ensemble sous agitation à 4°C pendant 24 heures. Les solvants sont ensuite évaporés sous pression réduite et le résidu chromatographie sur silice (toluène-éthanol 8:3). On récupère 10,8 mg (2,54 mmol) d'acide carboxylique.
Rendement 39 % ; Rf: 0,06 [SiO₂, toluène-éthanol (8:2)].

Voie B : Oxydation au permanganate de potassium

On dissout 145 mg (0,353 mmol) de formyl-4 β-carboline précédente dans un mélange contenant 30 ml d'acétone et 6 ml d'eau. On additionne 139 mg (0,879 mmol) de permanganate de potassium préalablement dissous dans 2 ml d'eau et on place l'ensemble sous agitation, à température ambiante pendant 3 heures. On ajoute alors un léger excès de formaldéhyde dans le mélange réactionnel et on agite jusqu'à disparition complète de la couleur violette du permanganate. Le dioxide de manganèse formé et éliminé par filtration sur filtre de verre fritté, rincé à l'acétone et le filtrat évaporé sous pression réduite. Le résidu de couleur jaune est repris avec du dichlorométhane et placé à 4°C pendant 2 heures. Le solide blanc qui a précipité dans ce milieu est filtré puis lavé à l'éthanol. On isole ainsi 127 mg (0,297) d'acide carboxylique attendu sous la forme d'une poudre blanche très fine.
Rendement 84 %.

### Etape 2 : Préparation du N-méthyl N-éthyléthoxycarbonyl-(éthoxy-carbonyl-9 N-tert butoxycarbonyl-4) β-carboline carboxamide-3.

L'acide de l'étape 1 (127 mg ; 0,30 mmol) est mis en suspension dans 20 ml de tertiobutanol distillé. L'ensemble est placé sous courant d'azote puis on y introduit 53 µl de triéthylamine distillée (0,39 mmol) et 77 µl de diphénoxyphosphorylazide (0,36 mmol). Le mélange réactionnel est alors porté à ébullition pendant 9 heures et agité à température ambiante pendant 20 heures. Après évaporation des réactifs et solvants sous pression réduite et chromatographie du résidu sur silice (toluène-éthanol, 8:1), on isole 38,2 mg (0,08 mmol) du produit attendu.
Rendement : 25 % ; Rf : 0,29 [SiO₂, toluène-éthanol (8:1)].

### Etape 3 : préparation de la N-éthyléthoxycarbonyl-(éthoxycarbonyl-9 amino-4) β-carboline-carboxamide

La β-carboline obtenue à l'étape 2 (50 mg : 0,1 mmol) est dissoute dans 3 ml de dichlorométhane anhydre. On ajoute 19 µl (0,2 mmol) d'acide trifluoroacétique à la solution et on place l'ensemble sous agitation, à température ambiante pendant 18 heures. Après cette période, on neutralise l'excès d'acide trifluoroacétique à l'aide de bicarbonate de sodium solide. Le mélange réactionnel est alors filtré et évaporé sous pression réduite. Le résidu chromatographié sur silice (toluène-éthanol, 8:0,2) conduit à l'amine attendue (6 mg) sous la forme d'un solide blanc.
Rendement : 15 % ; Rf : 0,10 [SiO₂, toluène-éthanol (8:1)].

### Etape 4 : Préparation du dihydro-1,12 éthoxycarbonyl-6 méthyl-2 indolo [3',2' : 4,5] pyrido [2,3-f] 1H, 3H, 6H diazépine-1,4 dione-3,12

L'acide obtenue à l'étape 1 (68 mg : 0,16 mmol) est mis en suspension, sous azote, dans 6 ml de toluène sec et l'ensemble est placé sous agitation à température ambiante. On y introduit 45 µl (0,21 mmol) de diphénoxyphosphorylazide, 33 µl (0,24 mmol) de triéthylamine distillée et on maintient l'agitation jusqu'à disparition complète du produit de départ (C.C.M. : SiO₂, toluène-éthanol, 8:2). Les réactifs et solvants sont alors évaporés sous pression réduite et le résidu solide, repris dans un mélange d'acide acétique (3 ml) et d'eau (3 ml). Le mélange réactionnel est porté à reflux pendant 1 h 30 mn. Après cette période, on dilue le milieu à l'acétate d'éthyle et on le neutralise à l'aide d'une solution aqueuse de bicarbonate de sodium (10 %). Après extraction, la phase organique est séchée, filtrée puis évaporée pour conduire à une huile (50 mg) que l'on chromatographie sur silice (toluène-éthanol, 8:2). On obtient la β-carbolinodiazépinone attendue (10,4 mg : 0,03 mmol) sous la forme d'un solide blanc.
Rendement : 12 % ; Rf : 0,30 [SiO₂, toluène-éthanol (8:2).

### Etape 5 : préparation du dihydro-1,12 méthyl-2 indolo [3',2' : 4,5] pyrido [2,3-f] 1H, 3H, 6H diazépine-1,4 dione-3,12 (composé de formule III où R₂ = R₆ = H, X = NCH₃).

L'hybride obtenu à l'étape 4 (10 mg ; 0,028 mmol) est dissous dans 2 ml d'éthanol contenant une quantité catalytique de sodium. La solution réactionnelle est placée sous agitation, à température ambiante, pendant 2 h 30 mn (disparition totale du produit de départ). Le mélange est alors neutralisé avec une petite quantité d'acide acétique puis les réactifs et solvants sont évaporés sous pression réduite. Le résidu huileux est repris au dichlorométhane ; le précipité résultant est filtré puis rincé à l'eau pour éliminer les sels inorganiques. On isole, après séchage, 5 mg (0,018 mmol) de l'hybride déprotégé (composé de formule III où R₂ = R₆ = H, X = NCH₃) sous la forme d'un solide blanc.
Rendement : 64 % ; F = 260°-270°C avec décomposition.

Les exemples ci-dessous illustrent les propriétés biologiques des composés selon l'invention.

### ACTIVITE BIOLOGIQUE

L'affinité de ces nouveaux ligands vis-à-vis du récepteur des benzodiazépines a été déterminée par des tests de liaison in vitro en utilisant des préparation de membranes corticales de cerveaux de rats (selon la méthode de Rehavi et coll. Eur. J. Pharmacol. 78, 353, 1982. Ces affinités, indiquées par les CI₅₀ (la concentration de substance nécessaire pour déplacer 50 % du ³H-flunitrazépam de ses sites de reconnaissance sur le récepteur des benzodiazépines), se situent à des concentrations proches de la nanomole (cf. tableau ci-après). Toutefois, une molécule se distingue, l'hydroxyméthylδ-lactone R, S- 5, qui possède une affinité de 0,18 nM. Cette affinité est 20 fois supérieure à celle de la β-CCE (4 nM) et 100 fois supérieure à celle du diazépam (20 nM). La lactone précitée représente donc la β-carboline la plus affinée pour le récepteur des benzodiazépines connue a ce jour.

Le profil pharmacologique a ensuite été déterminé.

L'effet de la lactone sur le récepteur des benzodiazépines exprimé a d'abord été exprimé, à partir de l'ARN messager correspondant, dans l'oocyte de Xénope. On a observé que, lorsque la lactone (10⁻⁶ M) est administrée simultanément avec une solution d'acide gammaaminobutyrique (GABA) à 10⁻⁵ M, il n'y a ni augmentation ni diminution du courant provoqué dans l'oocyte par cette dernière molécule. La lactone se comporte donc, dans ce test, comme un antagoniste du récepteur des benzodiazépines. De plus, la lactone (10⁻⁶ M) inhibe, de façon significative, l'augmentation du courant provoquée par un agoniste du récepteur tel que le diazépam (10⁻⁶ M).

Les propriétés antagonistes de la lactone ont également été vérifiées in vivo chez la souris. Administré aux souris, le dérivé (5 mg/kg, sc) ne produit aucun effet sur leur comportement, n'étant ni convulsivant ni sédatif. Par contre, la sédation provoquée par le diazépam (7,5 mg/kg, sc) est complètement empêchée par l'administration du composé (5 ou 10 mg/kg sc).

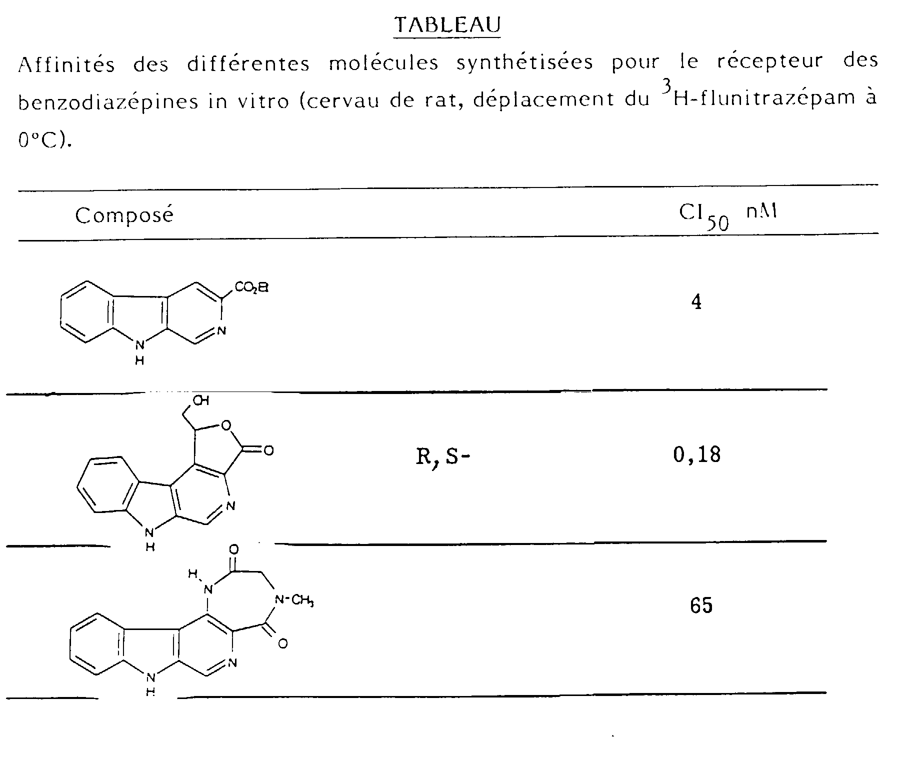

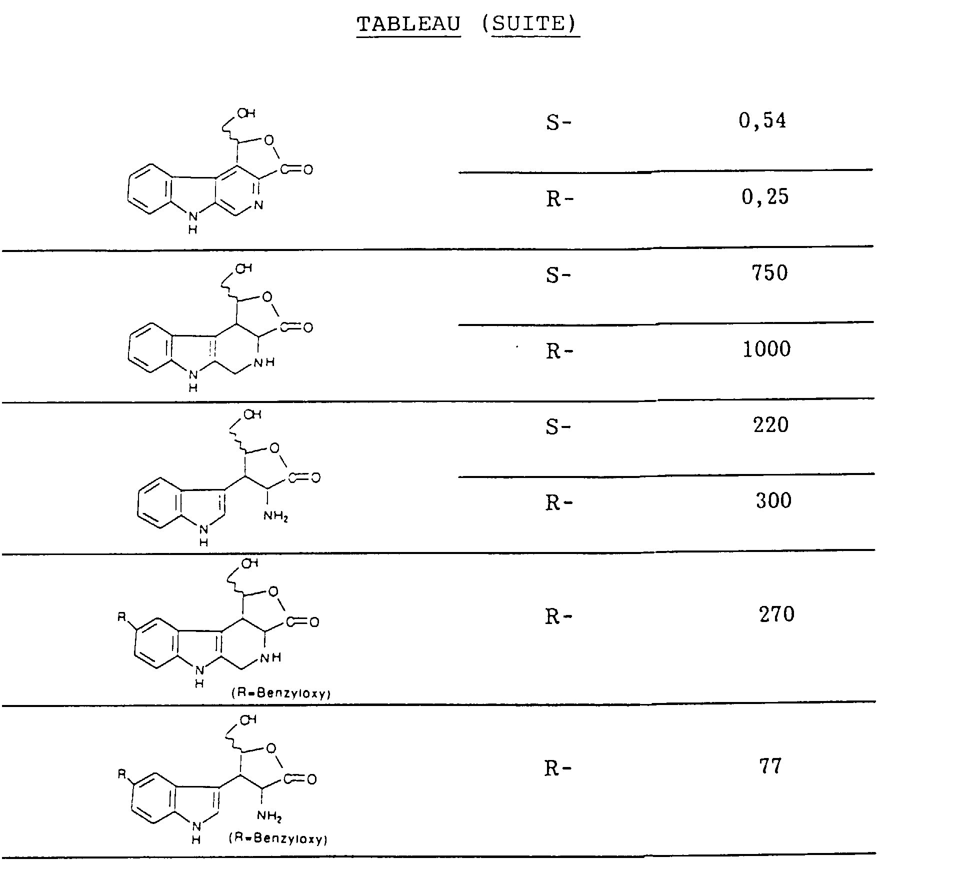

## Revendications

1. Composés de formule: 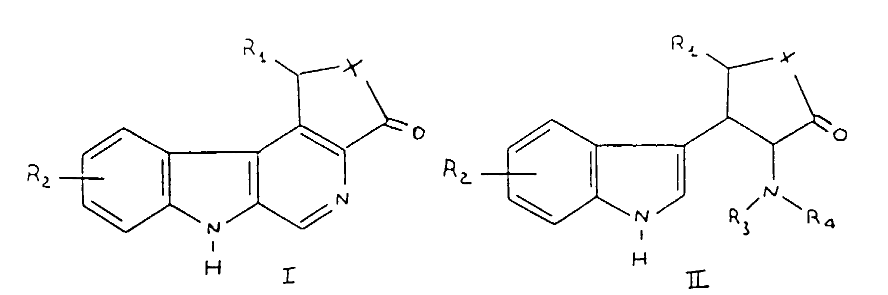 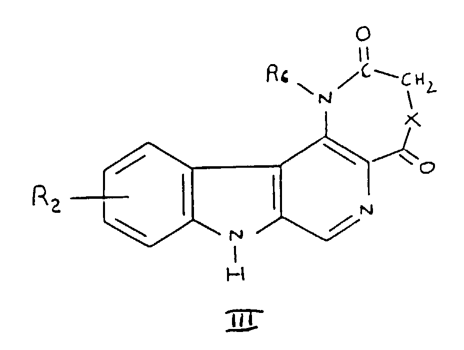 dans lesquelles :
X est l'atome d'oxygène, de soufre ou un radical divalent NR₅
R₁ est choisi parmi les radicaux alkyl substitués en α par un radical hydroxy ou alcoxy,
R₂ est un atome d'hydrogène ou un radical choisi parmi les radicaux alkyl, hydroxy, alcoxy, acyloxy, benzyloxy,
R₃, R₄, R₅ et R₆ sont choisis parmi l'atome d'hydrogène ou un radical alkyle éventuellement substitué par un radical hydroxy, alcoxy, alkoxycarbonylméthyle,
ainsi que les dérivés tétrahydro de β-carboline correspondants et les sels ou dérivés d'ammonium quaternaires de ces composés pharmaceutiquement acceptables et leurs formes optiquement actives.

2. Composés selon la revendication 1, caractérisé en ce qu'ils répondent à la formule I :
dans laquelle X est l'atome d'oxygène ou le radical divalent N-R₅ et R₁, R₂ ont la même signification que dans la revendication 1.

3. Composés selon la revendication 2, caractérisés en ce que R₁ est le radical hydroxyméthyle.

4. Composés selon la revendication 2, caractérisés en ce que R₂ est l'atome d'hydrogène.

5. Composés selon la revendication 1, caractérisés en ce qu'ils répondent à la formule Il dans laquelle les radicaux R₃ et R₄ sont l'atome d'hydrogène et X est l'atome d'oxygène ou un radical divalent 〉N-R₅, R₁ et R₂ ont la même signification que dans la revendication 1.

6. Composés selon la revendication 5, caractérisés en ce que R₁ est le radical hydroxyméthyle.

7. Composés selon la revendication 6, caractérisés en ce que R₂ est l'atome d'hydrogène.

8. Composés selon la revendication 1, caractérisés en ce qu'ils répondent à la formule : 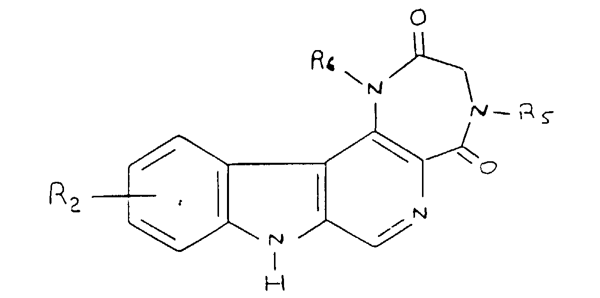 dans laquelle :
R₂ a la même signification que dans la revendication 1,
R₅, R₆ sont choisis parmi l'atome d'hydrogène ou un radical alkyle.

9. Composés selon la revendication 1 ou 8, caractérisés en ce que R₂ est l'atome d'hydrogène.

10. Composés tétrahydro de formule : 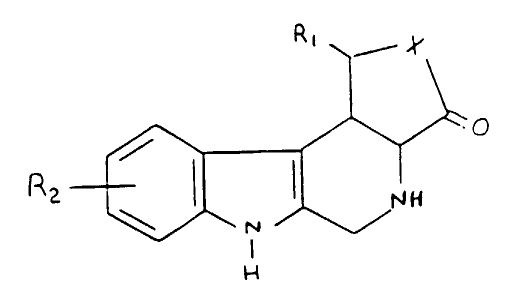 dans laquelle les substants R₁, R₂, X ont la même signification que dans l'une des revendications 1 à 5.

11. Composés selon l'une des revendications précédentes, caractérisés en ce qu'il sont choisis parmi les composés suivants :
- Dihydro-1,12 méthyl-2 indolo [3',2':4,5] pyrido [2,3-f] 1H, 3H, 6H diazépine-1,4 dione-3,12 ;
- Hydroxyméthyl-10 butanolide (2,3-b)-β-carboline,
- 3-hydroxyméthyl-4-(5-benzyloxy-3-indolyl)-5-amino-gamma-lactone.

12. Composés selon la revendication 11, caractérisés en ce qu'ils répondent à la formule :

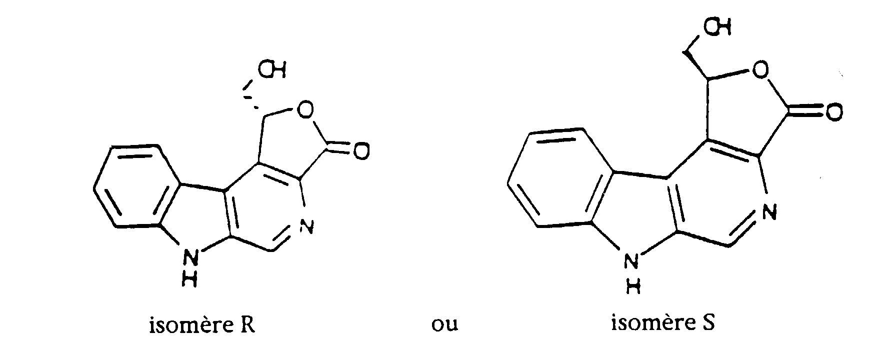

13. Médicament consistant en un composé de formule I ou Il elon l'une des revendications 1 à 12.

14. Composition pharmaceutique contenant au moins un médicament selon la revendication 13 et un support acceptable.

## Claims

1. Compounds of formula: 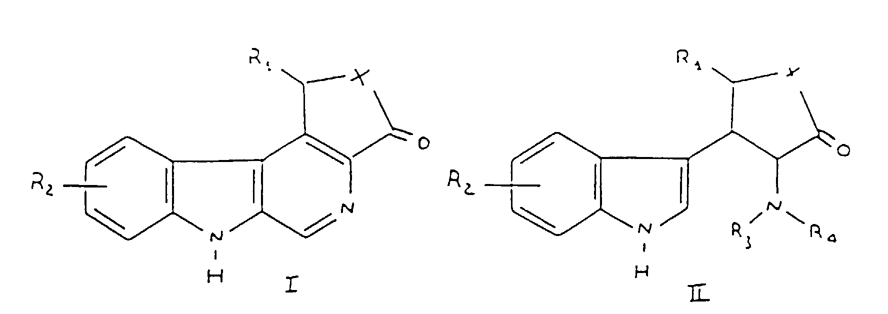 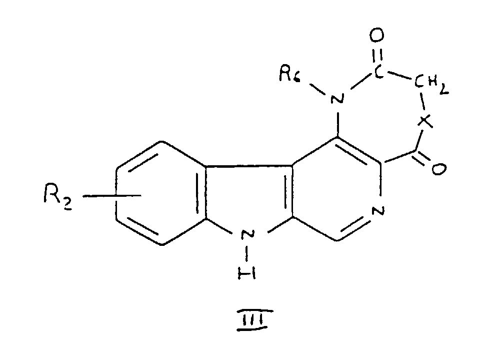 in which:
X is an oxygen or a sulphur atom or a divalent radical NR₅,
R₁ is chosen from alkyl radicals substituted in α by a hydroxyl or alkoxy radical,
R₂ is a hydrogen atom or a radical chosen from alkyl, hydroxyl, alkoxy, acyloxy or benzyloxy radicals,
R₃, R₄, R₅ and R₆ are chosen from a hydrogen atom or an alkyl radical optionally substituted by a hydroxyl, alkoxy or alkoxycarbonylmethyl radical,
as well as the corresponding tetrahydro derivatives of β-carboline and the quarternary ammonium salts or derivatives of these pharmaceutically acceptable compounds and their form optically active.

2. Compounds according to Claim 1, characterized in that they are of the formula I:
in which X is an oxygen atom or the divalent radical N-R₅, and R₁ and R₂ have the same meaning as in Claim 1.

3. Compounds according to Claim 2, characterized in that R₁ is a hydroxymethyl radical.

4. Compounds according to Claim 2, characterized in that R₂ is a hydrogen atom.

5. Compounds according to Claim 1, characterized in that they are of the formula II in which the radicals R₃ and R₄ are a hydrogen atom and X is an oxygen atom or a divalent radical 〉N-R₅, R₁ and R₂ have the same meaning as in Claim 1.

6. Compounds according to Claim 5, characterized in that R₁ is a hydroxymethyl radical.

7. Compounds according to Claim 6, characterized in that R₂ is a hydrogen atom.

8. Compounds according to Claim 1, characterized in that they are of the formula: 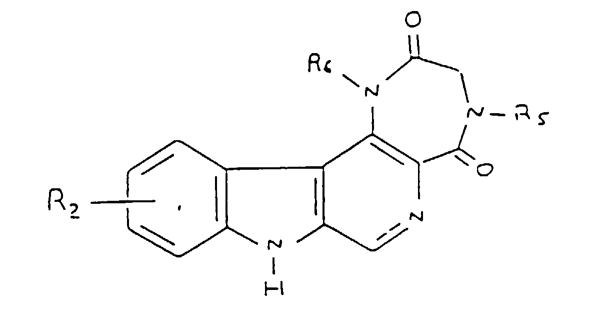 in which:
R₂ has the same meaning as in Claim 1,
R₅ and R₆ are chosen from a hydrogen atom or an alkyl radical.

9. Compounds according to Claim 1 or 8, characterized in that R₂ is a hydrogen atom.

10. Tetrahydro compounds of formula: 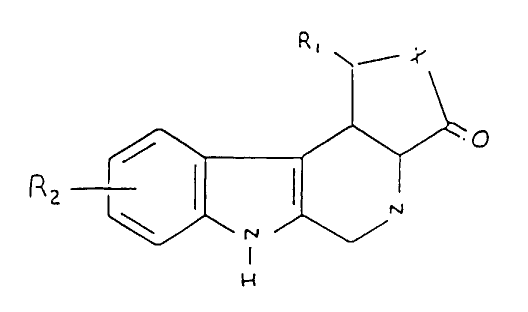 in which the substituents R₁, R₂ and X have the same meaning as in one of Claims 1 to 5.

11. Compounds according to one of the preceding claims, characterized in that they are chosen from the following compounds:
- 1,12-dihydro-2-methylindolo[3',2':4,5] pyrido-[2,3-f]-1H,3H,6H-1,4-diazepine-3,12-dione;
- 10-hydroxymethylbutanolide [2,3-b]-β-carboline,
- 3-hydroxymethyl-4-(5-benzyloxy-3-indolyl)-5-amino-gamma-lactone.

12. Compounds according to claim 11, characterized in that they are of the formula :

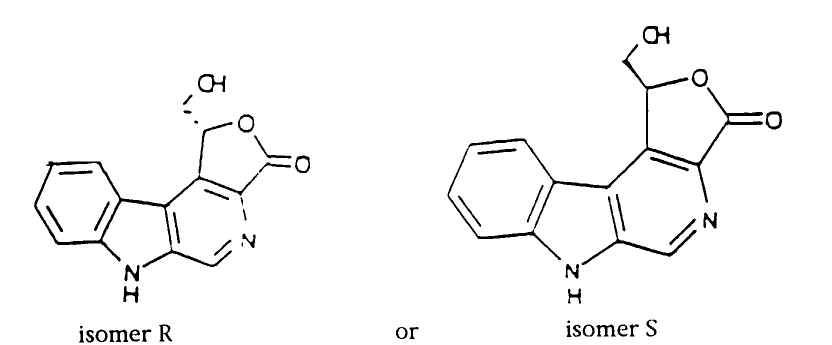

13. Drug consistinf of a compound of formula I or II according to one of Claims 1 to 12.

14. Pharmaceutical composition containing at least one drug according to Claim 13 and an acceptable carrier.

## Patentansprüche

1. Verbindungen der Formel 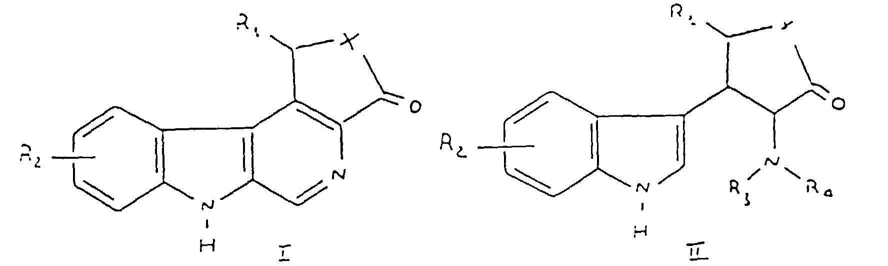 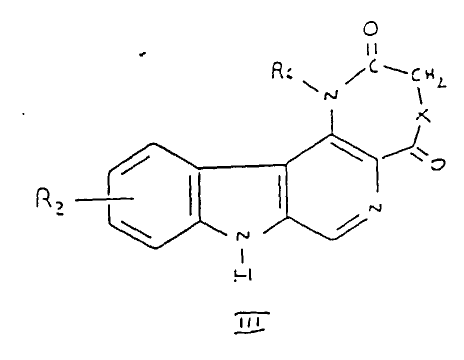 wobei:
X ein Sauerstoffatom, ein Schwefelatom oder ein divalenter NR₅-Rest ist
R₁ ausgewählt wird aus Alkylresten, die mit einem Hydroxy- oder Alkoxyrest substituiert sind,
R₂ ein Wasserstoffatom oder ein Rest ist, der ausgewählt wird aus Alkyl-, Hydroxy-, Alkoxy-, Acyloxy-, Benzyloxyresten,
R₃, R₄, R₅ und R₆ ausgewählt werden aus einem Wasserstoffatom oder einem Alkylrest, der gegebenenfalls substituiert ist mit einem Hydroxy-, Alkoxy-, Alkoxycarbonylmethylrest,
und die entsprechenden pharmazeutisch verträglichen Tetrahydro-b-carbolinderivate und die Salze oder quaternären Ammoniumderivate dieser Verbindungen und ihre optische aktive formen.

2. Verbindungen nach Anspruch 1, dadurch charakterisiert, daß sie der Formel I entsprechen:
wobei X ein Sauerstoffatom oder ein divalenter N-R₅-Rest ist und R₁, R₂ dieselbe Bedeutung haben wie in Anspruch 1.

3. Verbindungen nach Anspruch 2, dadurch charakterisiert, daß R₁ ein Hydroxymethylrest ist.

4. Verbindungen nach Anspruch 2, dadurch charakterisiert, daß R₂ ein Wasserstoffatom ist.

5. Verbindungen nach Anspruch 1, dadurch charakterisiert, daß sie der Formel II entsprechen, in der die Reste R₃ und R₄ ein Wasserstoffatom sind und X ein Sauerstoffatom oder ein divalenter NR₅-Rest ist, und R₁ und R₂ dieselbe Bedeutung haben wie in Anspruch 1.

6. Verbindungen nach Anspruch 5, dadurch charakterisiert, daß R₁ ein Hydroxymethylrest ist.

7. Verbindungen nach Anspruch 6, dadurch charakterisiert, daß R₂ ein Wasserstoffatom ist.

8. Verbindungen nach Anspruch 1, dadurch charakterisiert, daß sie der Formel: 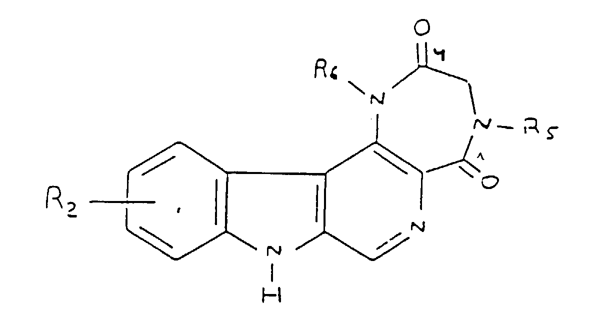 entsprechen, wobei:
R₂ dieselbe Bedeutung hat wie in Anspruch 1,
R₅, R₆ ausgewählt werden aus einem Wasserstoffatom oder einem Alkylrest.

9. Verbindungen nach Anspruch 1 oder 8, dadurch charakterisiert, daß R₂ ein Wasserstoffatom ist.

10. Tetrahydro-Verbindungen der Formel: 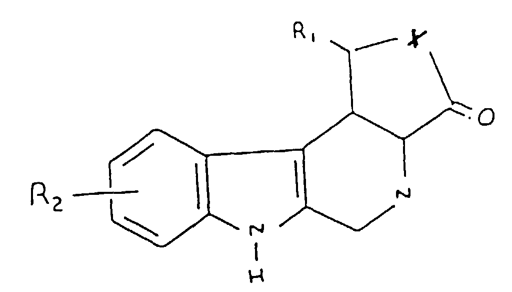 wobei die Reste R₁, R₂, X dieselbe Bedeutung haben wie in den Ansprüchen 1 bis 5.

11. Verbindungen nach einem der vorstehenden Ansprüche, dadurch charakterisiert, daß sie ausgewählt werden aus den folgenden Verbindungen:
- 1,12-Dihydro-2-methyl-[3',2':4,5]indolo[2,3-f]pyrido-1H,3H,6H-1,4-diazepin-3,12-dion;
- 10-Hydroxymethyl-butanolid(2,3-b)-β-carbolin;
- 3-Hydroxymethyl-4-(5-benzyloxy-3-indolyl)-5-amino-gamma-lacton.

12. Verbindungen nach Anspruch 11, dadurch charakterisiert, daß sie der formel

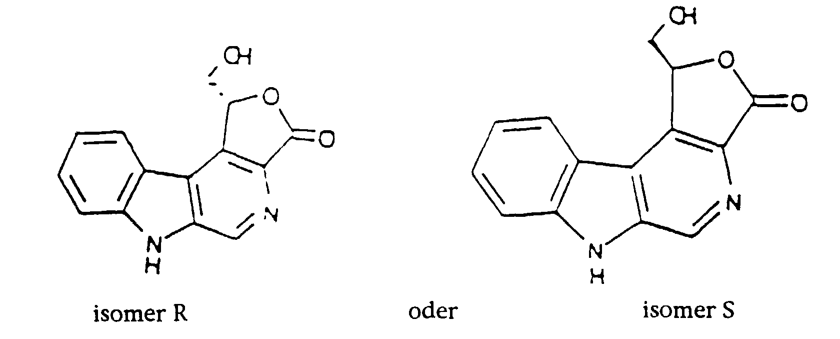

13. Medikament, bestehend aus einer Verbindung der Formel I oder II nach einem der Ansprüche 1 bis 12.

14. Pharmazeutische Zusammensetzung, enthaltend mindestens ein Medikament nach Anspruch 13 und einen verträglichen Träger.
